# EUROPEAN PATENT APPLICATION

(11) **EP 4 129 237 A1**
(43) Date of publication of application: **08.02.2023**
(21) Application number: 21315137.6
(22) Date of filing: 06.08.2021
(51) Int. Cl.: A61F 2/24

(54) **IMPROVEMENTS RELATING TO PROSTHETIC VALVES**

(71) Applicant: Epygon SAS, 13100 Aix-en-Provence (FR)
(72) Inventor: Ferraro, Mauro, 10138 Torino (IT); Cennamo, Tiziana, 13044 Crescertino (IT); Valerio, Lorenzo, 21052 Busto Arsizio (IT); Scorsin, Marcio, Jilin 130117 (CH)
(74) Representative: Hepp Wenger Ryffel AG

(57) **Abstract**

A prosthetic valve (10) comprising a frame (12) and a valve component (14), the frame and valve component being collapsible to a collapsed configuration for introduction to an implantation site, and expandable to an expanded configuration for implantation,
the frame having an interior channel housing substantially the valve component, the valve component comprising a valve member (16) of biological tissue,
the valve member comprising a leaflet body (18) capable of flexing between open and closed conditions, the leaflet body (18) forming a primary leaflet of the valve component such that the leaflet body spans at least a majority of interior channel at least in the closed condition of the leaflet body, the leaflet body including a free edge (20), at least one end (22) of the free edge being supported at an attachment position (24),
the valve component further comprising a patch (28) of flexible reinforcing material in face-to-face contact with a leaflet body portion that flexes, and extending to the attachment position for reinforcing the valve member near and at the attachment position.

## Description

### Field of the Invention

The present invention relates to the field of prosthetic valves for transcatheter delivery, especially but not exclusively cardiac valves, for example, mitral valves. Some embodiments relate to prostheses having a single or primary valve leaflet.

### Background to the Invention

Many different types of transcatheter cardiac valve prostheses have been proposed. One example is a tri-leaflet valve using three relatively small leaflets that collapse inwardly towards the center of the valve conduit, to thereby close the valve and prevent reverse flow. Although some characteristics of a tri-leaflet valve mimic the motion of a semi-lunar native valve, for example, a native aortic valve, there may be differences in valve hemodynamics compared to a native atrio-ventricular valve such as a mitral valve.

WO-A-2013/160439 and WO-A-2015/135883 describe promising alternative designs of prosthetic valve including a ring-shaped anchoring structure supporting a mono-leaflet valve. The structure includes a support wall portion at which a root end of the single valve leaflet is connected, and a complementary wall portion opposite the support wall portion which supports a coaptation surface adapted to be sealingly engaged by a free end of the valve leaflet when in its closed condition. A mono-leaflet valve can provide different valve hemodynamics from a tri-leaflet valve, and reduce the quantity of leaflet material within the conduit, thereby contributing to size reduction for implantation. However, a mono-leaflet valve also results in different distribution of closure forces on the valve and structure.

In WO-A-2013/160439, the free end of the valve leaflet is connected to the support wall portion by means of traction members dimensioned in such a way that the free end of the valve leaflet is stopped at the coaptation surface. Example traction members have the form of traction tethers similar to native mitral chordae tendineae for restraining the leaflet. In WO-A-2015/135883, arrangements of attachment tissue are described for reducing stresses in attachment sutures and for distribution of forces to the stent.

The single leaflet of a mono-leaflet valve is subjected to considerably greater forces and stresses than an individual leaflet of a tri-leaflet valve. The entire force exerted by blood pressure acting on the prosthesis during the cardiac cycle, and especially the closure force during systole in the case of a mitral valve, is borne by the single leaflet and its mounting, instead of being distributed as a lesser force on three leaflets.

It would be desirable to enhance certain characteristics of a valve such as a mono-leaflet valve, while maintaining the benefit of its different valve hemodynamics and reduction of leaflet material.

### Summary of the Invention

Aspects of the invention are defined in the claims.

Additionally or alternatively, one aspect of the invention provides a prosthetic valve comprising a frame and a valve component. The frame and valve component are collapsible to a collapsed configuration for introduction to an implantation site, and expandable to an expanded configuration for implantation.

The frame has an interior channel housing substantially the valve component. The valve component comprises a valve member of biological tissue. The valve member itself includes a leaflet body capable of flexing between open and closed conditions. The leaflet body forms a primary leaflet of the valve component such that the leaflet body spans at least a majority of the interior channel at least in the closed condition of the leaflet body.

The leaflet body includes a (e.g. mobile) free edge, at least one end of the free edge being supported at an attachment position. The valve component further comprising a patch (and/or panel) of flexible reinforcing material in face-to-face contact with a leaflet body portion that flexes, for reinforcing the valve member near and/or at the attachment position. For example, the patch may extend to the attachment position and/or to neighboring tissue, e.g. neighboring tissue of the valve component.

The attachment position may be defined at least partly by the frame and/or by tissue of the valve member and/or of the valve component (e.g. tissue other than the leaflet body tissue).

A further similar aspect, optionally in combination with the above, provides a prosthetic valve, comprising a frame and a valve component, the frame and valve component being collapsible to a collapsed configuration for introduction to an implantation site, and expandable to an expanded configuration for implantation.

The frame has an interior channel housing substantially the valve component, the valve component comprising a valve member of biological tissue. The valve member itself includes a leaflet body with a (e.g. mobile) free edge, and an extension integral with the leaflet body. The leaflet body is capable of flexing with respect to the extension between an open condition and a closed condition of the leaflet body, the leaflet body being a primary leaflet of the valve component spanning a majority of the interior channel, at least in the closed condition of the leaflet body.

The extension extends in a circumferential direction with respect to the frame, and the valve component further comprises a patch (and/or panel) of flexible reinforcing material overlapping partly and in face-to-face contact with the leaflet body and the extension, for providing a reinforced region of the valve member where the leaflet body meets the extension.

With such an arrangement in either aspect, the reinforcement material can reinforce the valve member in a region at an end of the free edge where stress concentration of a primary leaflet can be extremely high.

A primary leaflet has to withstand a majority of the entire force applied by blood pressure acting on the valve when closed. It has been appreciated that while certain stresses can be distributed along a fixed (e.g. belly) edge of the leaflet body, stress concentration is more pronounced at an extremity of the free edge. Stress concentrations at an extremity of the free edge are more complex than flexing along a fixed belly edge of the leaflet body, because the extremity of the free edge also bears more axial load as the valve reaches its closed condition. The combination of repeating axial loading and flexing results in greater risk of tearing than at the belly edge. The reinforcement can alleviate the stress concentration by distributing the forces in a greater thickness of material, and thereby reduce risk of a tear occurring in the valve member tissue. Should a tear begin near the end of the free edge, the patch of reinforcement material can support the valve member tissue, and lessen tear propagation.

Such stress concentration is particularly important for a primary leaflet because, in contrast to tri-leaflet valve, there is no natural counterbalancing effect of coaptation between equal leaflets. In order to satisfy durability norms, the prosthetic valve generally has to have an expected life of at least several years. For example, over a five-year period, the heart will beat around 200 million times, and the prosthetic valve is required to withstand the closure forces'and stresses 200 million times in order to maintain correct valve function, and hence maintain proper heart function.

Although the invention is not limited to an atrio-ventricular valve (for example, a mitral valve), the forces applied to such a valve are significantly higher than for a semi-lunar valve, for example, an aortic valve. An atrio-ventricular valve has to withstand the high blood pressure in the ventricle during systole. Also, an atrio-ventricular valve generally has a larger annulus size than a semi-lunar valve. A higher blood pressure exerted over a larger area results in significantly higher force on the valve component.

Such an arrangement can also provide support' for and reinforcement of the valve member without needing traction tethers at the free edge.

Avoiding traction tethers can'simplify construction of the valve. Avoiding traction tethers also enables a reduction in excess or slack material at the extremity of the valve during collapsing and loading into a delivery catheter.

In some embodiments, the free edge of the leaflet body includes first and second ends, the first end being supported at a first attachment position, and the second end being supported at a second attachment position. The valve component comprises a first patch of said reinforcement material at the first end, and a second patch of said reinforcement material at the second end, the first and second patches being distinct from one another. Optionally, the first and second patches are spaced apart from each other along the free edge, leaving a free portion of the free edge without the reinforcement material.

Additionally or alternatively, the free edge includes first and second ends, and the valve member comprises a first extension extending from the first end, and a second extension extending from the second end. The valve component comprises a first patch of said reinforcement material at the first end, and a second patch of said reinforcement material at the second end, the first and second patches being distinct from one another. Optionally, the first extension and the second extension are coupled to each other at a junction that is, for example, spaced from the leaflet body. Optionally, the first and second patches are spaced apart from each other along the free edge, leaving a free portion of the free edge without the reinforcement material.

Additionally or alternatively, the patch of reinforcing material extends adjacent to a first portion of the free edge of the leaflet body, the mobile free edge including a free portion without the patch of reinforcement material. Optionally, the first portion of the free edge is less than 30% of the total length of the free edge, optionally less than 25% of the total length of the free edge, optionally less than 20% of the total length of the free edge, optionally less than 15% of the total length of the free edge, optionally less than 10% of the total length of the free edge.

Such arrangements of first and second distinct patches and/or a free portion of the free edge can provide local reinforcement of the end(s) of the free edge, while leaving another portion of the free edge fully mobile without reinforcement. Positioning patches at the ends of the free edge can also beneficially increase the distribution of mechanical loads along the free edge. Compared to a leaflet without local reinforcement at the free edge ends, stress concentrations are reduced at the ends and/or the (e.g. central) unreinforced region of the free edge is more active mechanically. This is advantageous for a primary, leaflet, for example, assisting coaptation of the free edge especially in the region away from the ends of the free edge when closed or closing.

In some embodiments, the patch of reinforcing material extends adjacent to the or a first portion of the free edge of the leaflet body, and optionally does not project edgewise further than the free edge.

In some embodiments, the patch of reinforcing material is selected from:
material non-integral with the valve member and attached thereto in face-to-face contact with the leaflet body portion; or
material integral with the valve member and folded into face-to-face contact with the leaflet body portion.

In some embodiments, the patch of reinforcing material may be secured to the leaflet body portion so as to move in unison with the leaflet body portion.

In some embodiments, the patch of reinforcing material is attached to the leaflet body portion and/or to the valve member, by one or more of: stitching; and/or adhesive bonding; and/or fusion bonding.

In some embodiments, the patch of reinforcing material is made of, or comprises, one or more of:
biological tissue, for example, pericardial tissue; and/or
synthetic material, for example, any of woven or non-woven synthetic fabrics, or polymer films.

In some embodiments, the reinforcing material may be fibrous, the fibres having at least one alignment direction, and wherein an alignment direction of the fibres has a predetermined orientation with respect to a fibre orientation of the tissue of the leaflet body portion, the predetermined orientation optionally selected from: generally parallel; and/or generally perpendicular.

In some embodiments, the patch of reinforcing material comprises: a single layer of material; two layers of material; three layers of material; four or more layers of material.

In some embodiments, the patch of reinforcing material comprises plural layers of different material and/or plural layers of material having different material characteristics, for example, any of fibrous or non-fibrous, different material thickness, different fibre orientations, different directional characteristics.

Although certain features have been highlighted above and in the appended claims, protection is claimed for any novel feature or idea described herein and/or disclosed in the drawings, whether or not emphasis has been placed thereon.

### Brief Description of the Drawings

Fig. 1 is a schematic underside perspective view of a prosthetic valve;
Fig. 2 is a schematic plan view from an outflow end of the prosthetic valve of Fig. 1 in an open condition of the valve;
Fig. 3 is a schematic plan view similar to Fig. 2 but showing the valve in a closed condition;
Fig. 4 is a schematic plan view of tissue components of a valve component for the prosthetic valve;
Fig. 5 is a schematic plan view of a first sub-assembly of the tissue components of Fig. 4;
Fig. 6 is a schematic perspective view showing a detail of the sub-assembly of Fig. 5;
Fig. 7 is a schematic exploded view illustrating a relative orientation of a patch of reinforcing material in a one example;
Fig. 8 is a schematic exploded view similar to Fig. 7, illustrating a relative orientation in a second example; and
Fig. 9 is a schematic perspective view showing a final assembly of the tissue components of Figs. 4 and 5.'

### Detailed Description of Preferred Embodiments

Non-limiting embodiments are now described by way of example only, with reference to the accompanying drawings. In the drawings, the same or equivalent features are denoted by the same reference numerals, whether or not described in detail for each figure.

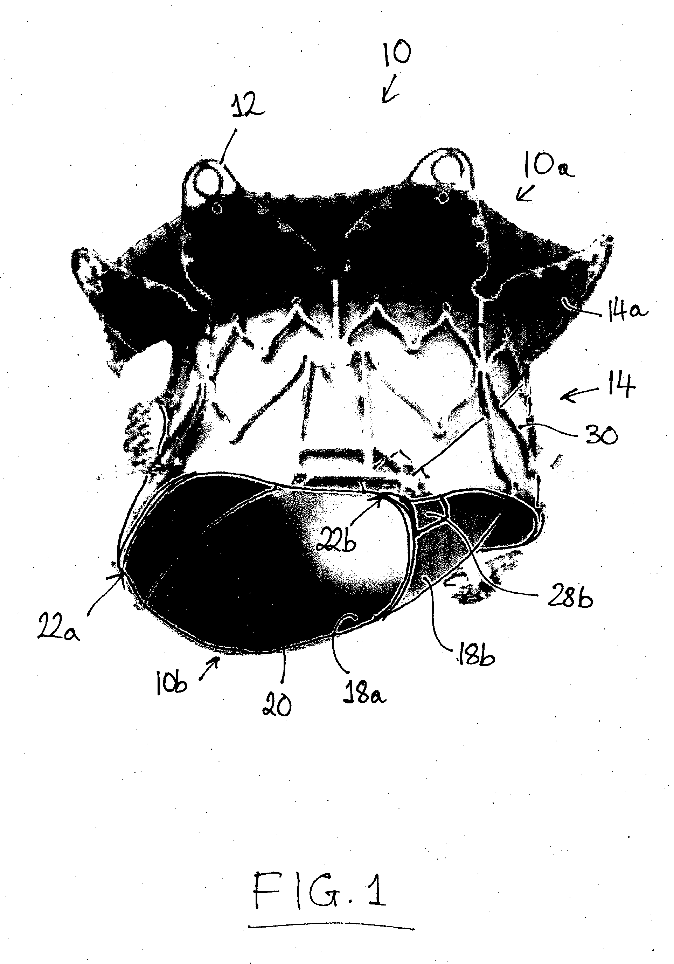

Referring to Fig. 1, a prosthetic valve 10 is illustrated in the form of a cardiac valve, optionally an atrio-ventricular valve, for example, a mitral valve. The prosthetic valve 10 generally comprises a frame 12 and a valve component 14. The frame 12 and valve component 14 are collapsible to a collapsed configuration (not shown) for introduction to an implantation site using, for example, a delivery catheter, and are expandable to an expanded configuration (Fig. 1) for implantation. The frame 12 defines an interior channel, and the valve component 14 is housed substantially within the interior channel. The prosthetic valve 10 has an inflow end 10a (the upper end in the orientation of Fig. 1), and an outflow end 10b (the lower end in the orientation of Fig. 1).

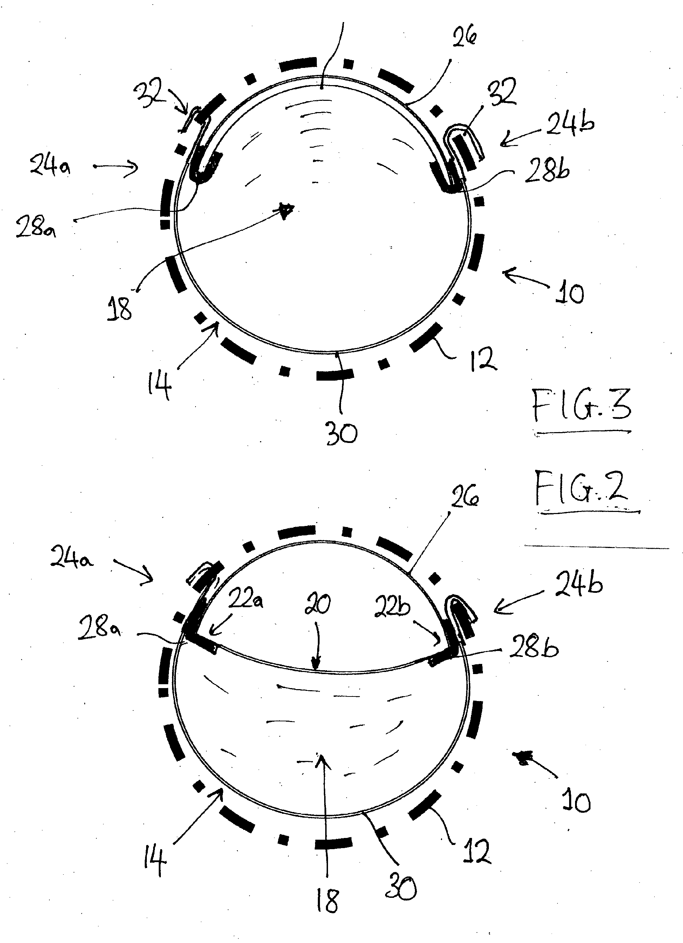

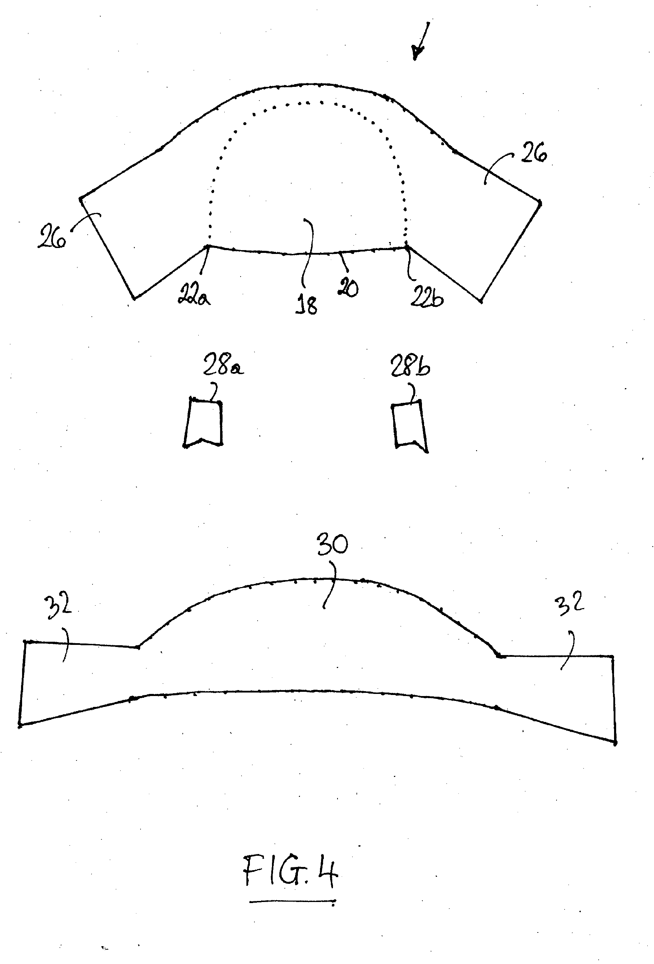

Referring to Figs. 2-3, but also to the component and sub-assembly views of Figs. 4-9, the valve component 14 comprises a valve member 16 of biological tissue. By way of example only, the tissue may be pericardial tissue, for example, porcine or bovine pericardial tissue. Although Figs. 2 and 3 illustrate the frame 12 and valve component 14 to have a generally circular shape, this is merely for schematic illustration. The shape may be non-circular, such as a D-shape, that may better fit the saddle shape of a native atrio-ventricular valve.

The valve member 16 includes a leaflet body 18 capable of flexing between an open condition (Fig. 2) and a closed condition (Fig. 3). In Fig. 4, the leaflet body 18 is delimited by broken lines. The leaflet body 18 forms a primary leaflet of the valve component 14, such that the leaflet body 18 spans at least a majority of the interior channel at least in the closed condition of the leaflet body 18 (Fig. 3). In the illustrated example, the primary leaflet may, for example, be a single leaflet of a mono-leaflet valve component. Alternatively, the valve component 14 may further comprise one or more secondary leaflets (not shown) that play a lesser role in the valving action of the valve component, and/or flex to a lesser extent and/or serve to cushion coaptation of the primary leaflet.

The leaflet body 18 comprises a free edge 20 having a first end 22a and second end 22b, at which the leaflet body 18 is supported at a respective attachment position 24a, 24b, respectively. The free edge 20 is the mobile edge of the leaflet body 18 that coapts with a complementary surface to block reverse flow through the valve. Various configurations of attachment positions 24 are envisaged. For example, the attachment positions 24 may correspond at least in part to attachment tissue extensions 26 of the valve member 16 on either side of the leaflet body 18. Generally, an attachment position 24 may be defined at least partly by the frame and/or by tissue of the valve member and/or of the valve component (e.g. tissue other than the leaflet body tissue).

A patch 28 (28a/28b) and/or panel (but referred to hereinafter as a patch) of flexible reinforcing material is attached in face-to-face contact with a region of the leaflet body 18 that flexes, and extends to at least one attachment position 24 (24a/24b), and/or to neighboring tissue (e.g. of the valve component), and/or to the extension 26. For example, the patch 28 overlaps a portion of the leaflet body 18 and a portion of the extension tissue 26. The patch 28 moves in unison with the portion of the leaflet body 18 that it overlaps. The reinforcement material serves to reinforce the valve member 16 at the end 22 of the free edge 20, near and/or at the attachment position. Reinforcement at this position can alleviate the risk of tissue damage from stress concentration at the end 22 of the free edge 20. Stress concentration at the end 22 of the free edge 20 may be different from that along a fixed belly edge of the leaflet body 18. Reinforcement can reduce risk of tear initiation and/or tear propagation at the free edge 20 resulting from stress concentrations. A primary leaflet has to withstand the majority (and in this example substantially all) of the force applied by blood pressure, especially at valve closing. The ability of the valve to continue to function for its expected operating life is dependent on withstanding such stress concentrations over millions of cycles (e.g. around 200 million cycles over five years), and maintaining the integrity of the tissue of the valve member 16.

In the illustrated example, two patches 28a and 28b of reinforcement material are illustrated, one for each end 22a, 22b of the free edge 20. The patches 28a and 28b are distinct from each other and, in this example, are spaced apart to leave a free portion 20a of the free edge 20 without reinforcement.

At least one patch 28, optionally each patch 28a, 28b, extends adjacent to a first portion 20b of the free edge 20 of the leaflet body, the free edge 20 including the free portion 20a without the patch of reinforcement material. The first portion 20b of the free edge (optionally individually, or optionally collectively) may be less than 30% of the total length of the free edge 20, optionally less than 25% of the total length of the free edge 20, optionally less than 20% of the total length of the free edge 20, optionally less than 15% of the total length of the free edge 20, optionally less than 10% of the total length of the free edge 20.

Spacing the patches 28a, 28b apart and/or provision of the free portion 20a of the free edge 20 enables the reinforcement to be localized where stress is most concentrated, near the ends 22a, 22b of the free edge 20. The free portion 20a can therefore remain relatively thin and conformable to enhance coaptation in the closed condition. It can also avoid introducing excessive material into the valve component 14 that might interfere with the ability to collapse the prostheses to a desirably small size. Not only do the patches 28a, 28b locally reinforce the ends 22a, 22b of the free edge, but the patches have an additional effect that mechanical loads are distributed more along the free edge, away from the ends 22a, 22b. This further reduces stress concentrations at the ends 22a, 22b, and makes the (e.g. central) unreinforced region 22c of the primary leaflet more active mechanically. Increased mechanical action at the central region 22c can enhance coaptation when the leaflet closes, and is particularly advantageous for the primary leaflet.

In the illustrated example, the (or each) patch 28 (28a, 28b) of reinforcing material extends adjacent to the first portion 20b of the free edge 20 of the leaflet body 18, and does not project edgewise substantially further than the free edge 20.

The leaflet body 18 has a coapting face 18a and an opposite non-coapting face 18b. The coapting face 18a is the face that contacts a seat-surface when the valve body 18 is in the closed configuration, to form a seal. In the illustrated example, the (or each) patch 28 (28a, 28b) of reinforcing material is attached to the non-coapting face 18b, so that the patch does not introduce irregularities on the coapting face 18a.

Various techniques for attaching the patch(s) 28 to the valve member 28 are envisaged. For example, the patch(s) 28 may be attached by one or more of: stitching; and/or adhesive bonding; and/or fusion bonding.

The patch(s) 28 of reinforcing material may be made of and/or comprise any suitable flexible material, for example: biological tissue, for example, pericardial tissue; and/or synthetic material, for example, any of woven or non-woven synthetic fabrics, or polymer films.

In the case of biological tissue, the reinforcing material may be the same type of biological tissue as the valve member 16, and optionally the same actual tissue. Whether or not the same type of tissue is used, biological tissue of the patch(s) 28 may have a tissue thickness that is smaller than, the same as, or greater than that of the valve member 16, depending on the degree of reinforcement desired.

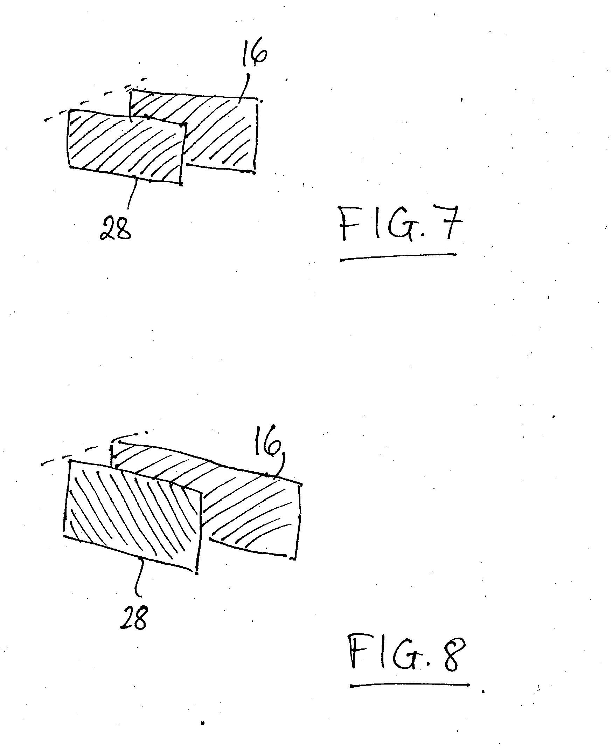

Referring to Figs. 7 and 8, in some embodiments, the reinforcing material of the patch(s) 28 may be fibrous, whether made of biological tissue or synthetic fabric, the fibres having at least one alignment direction, indicated by the diagonal lines in Figs. 7 and 8. The alignment direction of the fibres of the patch(s) 28 can have a predetermined orientation with respect to a fibre orientation of the tissue of the valve member 16 and/or the leaflet body 18. The predetermination orientation can be selected to provide a desired type of reinforcement. For example, referring to Fig. 7, the predetermined orientation may be generally parallel. Such orientation can preserve directional elasticity of, for example, biological tissue to permit material stretching. Alternatively, referring to Fig. 8, the predetermined orientation may be generally perpendicular. Such orientation can stiffen the biological tissue of the valve member 16 in the region of the patch(s) 28, by the patch(s) 28 and the valve member 16 having different directions in which each is more elastic. The patch(s) 28 resists stretching in the elasticity direction of the valve member 16, and the valve member 16 itself resists stretching in the elasticity direction of the patch(s) 28.

In some embodiments, at least one patch 28 of reinforcing material can comprise: a single layer of material; two layers of material; three layers of material; four or more layers of material.

In some embodiments, at least one patch 28 of reinforcing material can comprise plural layers of different material and/or plural layers of material having different material characteristics, for example, any of fibrous or non-fibrous, different material thickness, different fibre orientations, different' directional characteristics.

Referring to Figs. 4-6 and 9, one example technique for assembling the valve component 14 is illustrated. The sub-assembly figures are intended to illustrate the positioning of the patches 28a and 28b, and it will be appreciated that the order of some of the assembly steps may be modified, and/or several assembly steps may be combined into a single step.

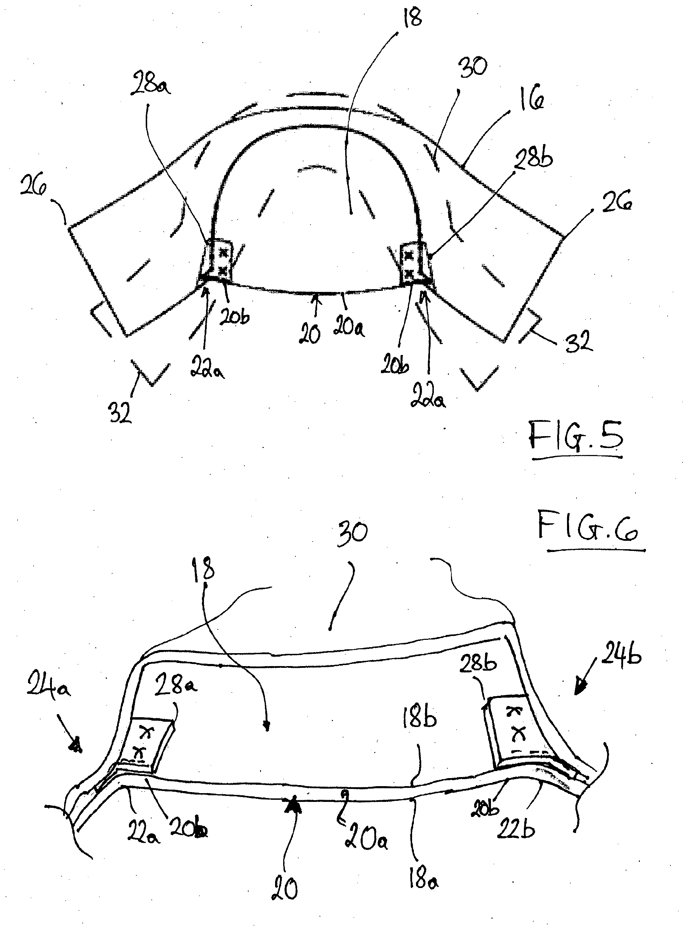

Referring to Fig. 4, the valve component 14 is made from the valve member 16, the patches 28a and 28b of reinforcement material, and an additional attachment tissue wall 30. Referring to Figs. 5 and 6, the patches 28a and 28b are attached to the valve member 16, for example, by suturing or by any other technique, for example, as described above. The example illustrates multiple suture patterns (e.g. a combination of at least two different suture patterns) to provide good reinforcement and resistance to tearing, especially at the free edge. The patches 28a and 28b are attached to the non-coapting face 18b of the leaflet body 18. Although in this example, the patches 28a and 28b are made of pieces of tissue that are separate from the valve member 16, in other embodiments, the patches 28a and 28b could be made as extensions of the valve member 16 and folded into face-to-face relation with the leaflet body 18 and with extensions 26.

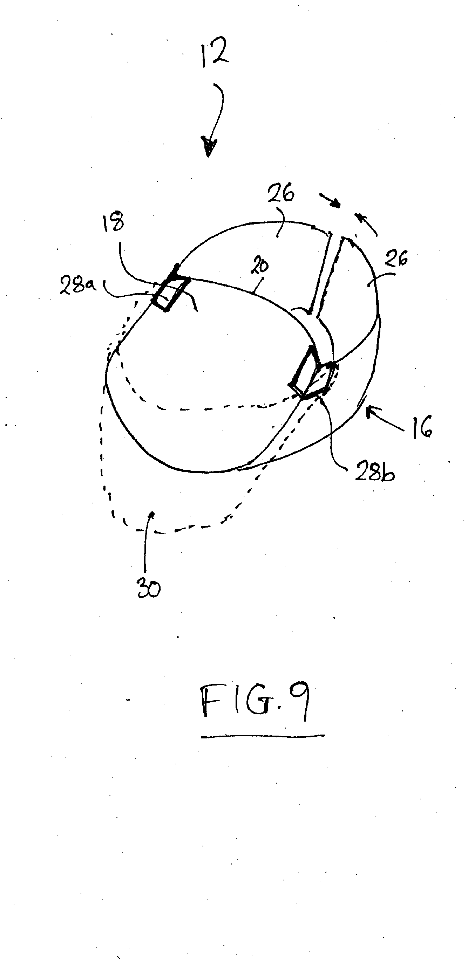

The sub-assembly is completed by the additional attachment tissue wall 30, which overlies the valve member 16 and the patches 28, and attaches to the arcuate belly edge around the leaflet body 18. In Figs. 5 and 9, the additional attachment tissue wall 30 is shown in a broken line in order to avoid concealing detail of the valve member 14. As best seen in Fig. 6, the additional tissue wall 30 is attached to portions of the patches 28a and 28b that extend beyond the ends 22a and 22b of the free edge 20, but the additional tissue wall 30 is not directly attached to portions of the patches 28a and 28b that are in face-to-face contact with the leaflet body 18.

Referring to Fig. 9, the extensions 26 are folded towards each other, and secured such that the valve member 16 has a tubular shape, one wall of which is formed by the leaflet body 18 inclined with respect to the extensions 26. The extensions 26 may, in use, constitute at least part of a wall (e.g. a posterior wall or an anterior wall) of the valve component 14, for example, for attachment to a portion of the frame 12 (e.g. to a posterior portion or an anterior portion, respectively). The extensions 26 may optionally also form a counter surface against which the leaflet body 18 coapts, or an additional patch and/or secondary leaflet may be added to the interior faces of the extensions to present a counter surface and/or cushion surface. The patches 28a and 28b can be seen in Fig. 8 on the non-coapting face 18b of the leaflet body 18, adjacent, to the ends 22a and 22b of the free edge 20, and extending in engagement with adjacent portions of the attachment tissue extensions 26.

Excess material 32 of the tissue wall 30 can be used to wrap around portions of the frame 10 corresponding to the attachment positions 24 described above.

In the illustrated example, the extensions 26 collectively form or form part of a posterior wall of the valve component 14, for example, for securing to a posterior portion of the frame 12. The additional wall 30 may form or form part of an anterior wall of the valve component 14, for example, for securing to an anterior portion of the frame 12. In other embodiments (not shown), the disposition may be reversed, such that the extensions 26 collectively form or form part of an anterior wall, and the additional wall 30 forms or.forms part of a posterior wall.

In a further example (not shown), instead of being sutured to the extensions 26, the reinforcement patches 28a/28b may be attached (e.g. sutured) to the additional wall 30.

In a yet further example (not shown), the additional wall 30 may extend substantially entirely around the periphery of the valve component 14, to provide both an anterior wall portion and a posterior wall portion. The extensions 26 may optionally be shortened compared to the illustrated example. The reinforcement patches 28a/28b may be attached (e.g. sutured) to the extensions 26 and/or to the additional wall 30. A slit may be provided in the additional wall 30 to allow material (e.g. the leaflet and/or the reinforcement patches) to pass through, for example, for attachment.

In a yet further example (not shown), first and second additional wall components (similar to wall 30) may be provided, one of the components forming or forming part of an anterior wall of the valve component, and the other component forming or forming part of a posterior wall of the valve component. The extensions 26 may be shortened compared to the illustrated form. The reinforcement patches 28a/28b may be attached to one or both of the first and second additional wall components, and/or to the extensions 26.

Other constructions of the valve component 14 may also be used.

Referring to Figs. 2 and 3, it can be seen that in the illustrated example, the material of the valve member 16 at the ends 22a and 22b of the free edge 20 bends as a hinge, as the leaflet body 18 flexes between the open condition (Fig. 2) and closed condition (Fig. 3). The patches 28a and 28b of reinforcement material extend around the exterior of the bend of the hinge, especially evident in the closed configuration. The patches 28a and 28b act at least partly in tension on the exterior of the bend of the hinge, and can additionally provide some traction effect to support the free edge 20 as the free edge reaches the closed condition.

The frame 12 may be configured like a stent, and comprise struts and/or cells shaped to permit the frame 12 to be compressed to its collapsed configuration, and expanded to the expanded configuration. The frame may at least partly self-expanding and made of shape memory alloy, for example, a Ni-Ti alloy, for example nitinol. Additionally or alternatively, the frame 12 may be forcibly expandable, for example, by using a dilation balloon.

Referring to Fig. 1, the inflow end 10a of the prosthesis may comprise a flared mouth. The valve component 12 may also have a flared inflow end 14a formed, for example, by a further tissue component (not shown separately).

It will be appreciated that the provision of one or more patches 28 of reinforcing material in accordance with principles described herein, can provide significant advantages in reinforcing an end of a free edge of a primary leaflet where stress concentrations can be particularly pronounced. This can enhance the durability of the prosthetic valve without adding too much additional or excess material, and without over-complicating the valve design.

It is emphasized that the above description is merely illustrative of a non-limiting embodiment of the invention, and that many equivalents and modifications may be used within the scope and/or principles of the present disclosure.

## Claims

1. A prosthetic valve (10) comprising a frame (12) and a valve component (14), the frame and valve component being collapsible to a collapsed configuration for introduction to an implantation site, and expandable to an expanded configuration for implantation,
the frame having an interior channel housing substantially the valve component, the valve component comprising a valve member (16) of biological tissue,
the valve member comprising a leaflet body (18) capable of flexing between open and closed conditions, the leaflet body (18) forming a primary leaflet of the valve component such that the leaflet body spans at least a majority of interior channel at least in the closed condition of the leaflet body, the leaflet body including a free edge (20), at least one end (22) of the free edge being supported at an attachment position (24),
the valve component further comprising a patch (28) of flexible reinforcing material in face-to-face contact with a leaflet body portion that flexes, and extending to the attachment position and/or to neighboring tissue, for reinforcing the valve member near and/or at the attachment position.

2. The prosthetic valve of claim 1, wherein the free edge (20) includes first and second ends (22a, 22b), the first end being supported at a first attachment position (24a), and the second end being supported at a second attachment position (24b), the valve component comprising a first patch (28a) of said reinforcement material at the first end, and a second patch (28b) of said reinforcement material at the second end, the first and second patches (28a, 28b) being distinct from one another.

3. A prosthetic valve (10), optionally according to any preceding claim, comprising a frame (12) and a valve component (14), the frame and valve component being collapsible to a collapsed configuration for introduction to an implantation site, and expandable to an expanded configuration for implantation,
the frame having an interior channel housing substantially the valve component, the valve component comprising a valve member (16) of biological tissue,
the valve member comprising a leaflet body (18) with a free edge (20), and an extension (26) integral with the leaflet body, the leaflet body capable of flexing with respect to the extension between an open condition and a closed condition of the leaflet body, the leaflet body (18) being a primary leaflet of the valve component spanning a majority of the interior channel, at least in the closed condition of the leaflet body, and the extension (26) extending in a circumferential direction with respect to the frame,
the valve component further comprising a patch (28) of flexible reinforcingmaterial overlapping partly and in face-to-face contact with the leaflet body (18) and the extension (26), for providing a reinforced region of the valve member where the leaflet body meets the extension.

4. The prosthetic valve of claim 3, wherein the free edge (20) includes first and second ends (22a, 22b), and the valve member comprises a first extension (26) extending from the first end, and a second extension (26) extending from the second end, the valve component comprising a first patch (28a) of said reinforcement material at the first end (22a), and a second patch (28b) of said reinforcement material at the second end, the first and second patches (28a, 28b) being distinct from one another.

5. The prosthetic valve of claim 4, wherein the first extension (26) and the second extension (26) are coupled to each other at a junction.

6. The prosthetic valve of any preceding claim, wherein the patch (28) of reinforcing material extends adjacent to a first portion (20b) of a free edge of the leaflet body, the free edge including a free portion (20a) without the patch of reinforcement material, optionally wherein the first portion (20b) of the free edge is less than 30% of the total length of the mobile free edge, optionally less than 25% of the total length of the mobile free edge, optionally less than 20% of the total length of the mobile free edge, optionally less than 15% of the total length of the mobile free edge, optionally less than 10% of the total length of the mobile free edge.

7. The prosthetic valve according to any preceding claim, wherein the patch (28) of reinforcing material extends adjacent to the or a first portion (20b) of the free edge (20) of the leaflet body, and optionally does not project edgewise further than the free edge (20).

8. The prosthetic valve according to any preceding claim, wherein the leaflet body (18) has a coapting face (18a) and a non-coapting face (18b), and wherein the or each patch (28) of reinforcement material is attached to the non-coapting face (18b).

9. The prosthetic valve of any preceding claim, wherein the patch (28) of reinforcing material is secured to the leaflet body portion (18) so as to move in unison with the leaflet body portion.

10. The prosthetic valve according to any preceding claim, wherein the patch (28) of reinforcing material is attached to the leaflet body portion and/or to the valve member, by one or more of:
stitching; and/or adhesive bonding; and/or fusion bonding.

11. The prosthetic valve according to any preceding claim, wherein the patch (28) of reinforcing material is made of, or comprises, one or more of:
biological tissue, for example, pericardial tissue; and/or
synthetic material, for example, any of woven or non-woven synthetic fabrics, or polymer films.

12. The prosthetic valve according to any preceding claim, wherein the reinforcing material is fibrous, the fibres having at least one alignment direction, and wherein an alignment direction of the fibres has a predetermined orientation with respect to a fibre orientation of the tissue of the leaflet body portion, the predetermined orientation optionally selected from: generally parallel; and/or generally perpendicular.

13. The prosthetic valve according to any preceding claim, wherein the patch (28) of reinforcing material comprises: a single layer of material; two layers of material; three layers of material; four or more layers of material.

14. The prosthetic valve according to claim 13, wherein the patch (28) of reinforcing material comprises plural layers of different material and/or plural layers of material having different material characteristics, for example, any of fibrous or non-fibrous, different material thickness, different fibre orientations, different directional characteristics.

15. The prosthetic valve of any preceding claim, wherein the prosthetic valve (10) is one or more selected from: an atrio-ventricular valve; a mitral valve; a mono-leaflet valve.
